(19) Europäisches Patentamt
European Patent Office
Office européen des brevets

(11) Publication number: **0 251 680 B1**

# (12) EUROPEAN PATENT SPECIFICATION

(45) Date of publication of patent specification: **15.04.92** (51) Int. Cl.⁵: **A61K 9/26**

(21) Application number: **87305625.3**

(22) Date of filing: **24.06.87**

## (54) Controlled release bioerodible drug delivery system.

(30) Priority: **25.06.86 US 878272**

(43) Date of publication of application:
**07.01.88 Bulletin 88/01**

(45) Publication of the grant of the patent:
**15.04.92 Bulletin 92/16**

(84) Designated Contracting States:
**AT BE CH DE ES FR GB GR IT LI LU NL SE**

(56) References cited:
**EP-A- 0 036 351**
**US-A- 3 981 303**
**US-A- 4 115 544**
**US-A- 4 179 497**

(73) Proprietor: **IOLAB, INC**
**500 Iolab Drive**
**Claremont California 91711(US)**

(72) Inventor: **Shell, John Weldon**
**952 Tournament Drive**
**Hillsborough, CA 94010(US)**

(74) Representative: **Jones, Alan John et al**
**CARPMAELS & RANSFORD 43 Bloomsbury Square**
**London, WC1A 2RA(GB)**

EP 0 251 680 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to controlled release drug delivery compositions and more particularly to drug dosage forms providing controlled release of selected therapeutic agents. Still more particularly, the invention relates to ocular dosage forms for controlled release of medications to the eye.

In supplying a drug or pharmaceutically active compound to bodily tissues, such as surgical sites, or bodily cavities, such as the eye, it is desirable to maintain a therapeutically effective concentration of the compound in the tissues without greatly exceeding that effective concentration. One area of medical therapy wherein the need for continuous and controlled administration of drugs is necessary is exemplified by medical treatment of ophthalmic conditions. For instance, eyedrops represent the most commonly used dosage form for ocular drugs. The time course of availability of a drug administered by eyedrops is characterized by an initial pulse-entry (which represents a transient overdose), followed by a rapid decline of drug concentration until the next administration (which represents a long period of under-dosing). Thus, in order for the eyedrops to provide enough drug to last for a reasonable period of time, an excessive amount must initially be administered. The side-effects of most ophthalmic drugs have been shown to be dose-related and to be associated with the pulse-entry.

Another problem associated with the use of eyedrops is due to the chemical instability of certain drugs when stored in aqueous solution (eyedrops). Further, certain otherwise useful ophthalmic drugs have very short biological halflives, and must therefore be administered very frequently. Finally, co-administration of certain useful drug combinations is not possible from eyedrop formulations, since the separate ingredients often require different frequencies of administration.

Precise control of the rate of drug release to the tissues, however, would provide an opportunity to overcome the above shortcomings. Controlled delivery would allow for avoidance of the pulse-entry, with which side-effects are associated, and therefore would eliminate or minimize these effects. By providing the drug delivery over an extended period of time, frequent administrations would not be necessary, thus enhancing patient compliance. Moreover, round-the-clock administration made possible by controlled delivery results in the maintenance of therapeutic levels of drug at times when patients are not likely to use eyedrops, e.g. the nighttime hours. Further, continuous controlled drug delivery overcomes the problem of rapid wash-out of drug by the tears, and it also allows for utilization of drugs that have very short biological halflives. In addition, controlled delivery allows for use of the optimum release rate for each of several ingredients of a drug combination, which overcomes the problem eyedrop combination products have if their ingredients require widely different frequencies of administration. Finally, a solid state drug delivery system would overcome the problem of storage due to solution instability of certain otherwise useful drugs, e.g. certain antibiotics, aspirin and epinephrine. Accordingly, a number of techniques have been devised to provide for a controlled continuous supply of medication to the eye by controlled release of the drug from a solid dosage form.

US-A-3,981,303 discloses an ocular dosage form comprising a multi-laminar structure wherein a drug to be administered to the eye is confined between polymeric membranes which permit diffusion of the drug therethrough. Over a period of time which may vary from a few hours to several days, the drug diffuses from the interior through the membranes and into contact with the ocular tissues. A key feature of the dosage form is that the polymeric membranes bioerode concurrently with, or at a point in time after, the dispensing of the drug. The membranes do not bioerode before the dispensing of the drug. While such has been found useful in the treatment of certain eye conditions, such as glaucoma, which require constant administration of medication, it has the disadvantages that it is relatively bulky and noticeable to the wearer and must be inserted and removed by the patient. The device also requires precise construction and sealing to minimize uncontrolled release of the drug, which requires great care and expense in its manufacture. In spite of such routine efforts, occasional leakage of these systems has been reported.

Attempts have been made to administer medication to the eye in the form of thin, solid, polymeric ocular inserts (lamellae) having the medication dispersed homogeneously within a hydrophilic polymer matrix. The drug is leached from the matrix by the tears and distributed through the eye by the flow of the tear fluid. These dosage forms have the disadvantage that typically a surge of drug is provided to the eye when the lamella is first inserted into the eye. For the treatment to last a reasonable period of time, this surge must be significant, and it is thus associated with significant drug side-effects. After the surge, the concentration of drug rapidly declines below the effective level as it is washed out of the eye by the flow of tears.

US-A-3,962,414 discloses a drug delivery device for the continuous and controlled administration of a therapeutically effective dosage of an eye drug over time using a thin, structured matrix of a bioerodible poly(lactic acid) polymer having dispersed therein microcapsules containing the drug. The drug release rate

is determined by the structure of the microcapsules, and the drug released from the microcapsules diffuses through the poly(lactic acid) matrix to the surface of the dosage form where it dissolves in the tear fluid and comes into contact with the tissues of the eye. The poly(lactic acid) matrix slowly bioerodes, releasing the microcapsules to the tear fluid. The particles are then washed away through the tear duct by the flow of tear fluid, since they are of sufficiently small size (less than 50 μm) to be washed from the eye. The matrix is eventually eroded, solublized and flushed from the eye. The rate of drug delivery tends to be rapid when the dosage form is first inserted into the eye and declines over time. Further, this dosage form, in common with the membrane controlled diffusion devices, remains as a foreign object in the eye for a long time, and may be perceived as uncomfortable by the patient. Sometimes, these relatively large dosage forms are extruded naturally from the eye with the consequence that the dosage form must be reinserted to continue the treatment regimen. The loss may not noticed by the patient, with the resulting serious consequence that the treatment is discontinued or interrupted. Re-insertion of an expelled system increases the risk of bacterial contamination of the eye.

US-A-4,115,544 discloses a fluid medium with suspended microparticles of a controlled-release matrix containing a drug. US-A-4 115 544 does not disclose microparticles suspended in a solid medium. Moreover, if the suspension has to be delivered to an eye in a controlled fashion, a specially designed container has to be used. The suspension of microparticles is administered to the eye in the same way as ordinary eye drops. However, the microparticles containing a drug were of sufficient size that they were found to remain within the conjunctival cul-de-sac, where they dispensed their medication over an extended period of time either by diffusion of the drug from the microparticle matrix or by the gradual bioerosion of the microparticles. Eventually, the microparticles were totally bioeroded and/or flushed from the eye by the flow of tear fluid. This technique has the drawbacks: that the microparticles begin release of a part of the drug while still in the aqueous medium of the container prior to use; that the administration of a liquid to the eye is less convenient than the use of a solid dosage form; and that accurate measurement of the dose is more difficult than with solid dosage forms.

The invention comprises a controlled release solid ocular drug dosage form which is bioerodible, which will not be subject to ejection from the eye and which will not cause discomfort to the patient. The invention is also a bioerodible solid drug dosage form that is to deliver a selected drug or biological agent to the ocular tissues within the site in a controlled fashion; and also extends to other surgical sites, such as arthroscopic sites and sites for glaucoma filtering procedures, for the same purpose.

According to the present invention, there is provided a composition for controlled release of a drug contained therein to a body fluid, said composition comprising an external matrix soluble in said body fluid, and microparticles dispersed in said external matrix, characterised in that: said microparticles comprise a drug dispersed within a bioerodible release rate controlling matrix and are capable of releasing the drug gradually to said body fluid; and the external matrix is substantially completely soluble in the body fluid within from 10 seconds to 30 minutes.

A bioerodible composition for controlled release of a drug to a body fluid according to the present invention therefore comprises: i) an external matrix rapidly soluble in the body fluid, and ii) bioerodible microparticles dispersed in the external matrix which contain the drug and are capable of releasing it gradually to the body fluids. The invention also comprises dosage forms containing the controlled release composition of this invention. In particular, the invention comprises an ocular dosage form for controlled release of a pharmaceutical compound to the tear fluid of a mammalian eye, which comprises i) an external matrix rapidly soluble in said tear fluid of the eye, and ii) bioerodible microparticles (or microcapsules) dispersed in the external matrix, the microparticles containing the pharmaceutical compound and being capable of releasing it gradually to the tear fluid, and being of a size to be retained in the eye long enough to provide an effective dose of the pharmaceutical compound to the eye prior to their ultimate dissolution.

The invention allows the use of a method of supplying a drug to a body fluid and thereby to tissues in contact with that body fluid which comprises contacting the body fluid with a dosage form comprised of the controlled release composition of the invention.

Objects of this invention include a controlled release drug dosage composition; such a composition for use in supplying a drug to tissues at a controlled rate; a controlled release dosage composition for controlled release of a drug to a body fluid and tissue in contact with the body fluid; a dosage form for controlled release of a drug to a body fluid; a composition for supplying a drug to tissues at a surgical site; an ocular dosage form containing microcapsules having controlled release characteristics for a drug or drugs contained therein; an ocular dosage form having an outer matrix which serves as a solid state carrier for particles which are unstable in an aqueous environment; an ocular dosage form having a rapidly dissolving outer matrix; an ocular dosage form which is comfortable to the user by reason of the rapid dissolution of its outer matrix after insertion into the eye; an ocular dosage form containing particles of a

controlled release drug composition of a size to be retained in the eye for a relatively long period of time; a sustained release ocular dosage form comprised of microparticles of a controlled release drug matrix which are small enough so that they are not ejected spontaneously from the eye; a controlled release ocular dosage form comprised of controlled release drug matrix particles which are small enough so that they are not perceived by the patient as foreign bodies; and a controlled release ocular dosage form containing microparticulate drug reservoirs which are bioerodible and ultimately soluble in the tear fluid.

Other objects are a controlled release solid ocular drug dosage form which is bioerodible, and which can be surgically implanted in ocular tissues without discomfort or toxic reaction to deliver an incorporated drug in controlled fashion to the tissues of the implantation site; an ocular dosage form having a rapidly dissolving outer matrix, which rapidly dissolves following surgical implantation, and which deposits thereby microparticles or microcapsules having controlled release characteristics for a drug or drugs contained therein; an ocular dosage form having microparticles of such composition that they are retained in the surgical site, in a functioning state, for a relatively long period of time; and a controlled release, surgically implantable, ocular dosage form containing microparticular drug reservoirs which are bioerodible and ultimately soluble in the tissue fluid.

In the accompanying drawings:

Figure 1 is a view, partly in front elevation and partly diagrammatic, of a human eye, illustrating the location of one dosage form embodiment of this invention immediately after insertion into the cul-de-sac of the eye;

Figure 2 is a side elevation cross section view of an eye illustrating the parts of the eye and the initial placement of the above dosage form embodiment of this invention therein;

Figures 3A, 3B and 3C illustrate various shapes of ocular dosage forms according to this invention, each partly cut away;

Figures 4, 5 and 6 are enlarged sectional views of a portion of dosage forms made from compositions of the invention showing three types of microparticle drug reservoirs according to the invention, some of the microparticles being shown partly cut away;

Figure 7 is an enlarged sectional view of a portion of a dosage form made from a composition of the invention showing another type of microparticle;

Figure 8 is a plot of the amount of drug released versus time as described in Example 13; and

Figure 9 is a plot of the amount of drug released versus time as described in Example 14.

The compositions of the present invention are adapted to be formed into dosage forms which can be inserted into body cavities to deliver drugs to these cavities and tissues in close proximity. The drug is contained in microparticles which, after they are inserted, release the drug to the body fluids present in body cavities. Body cavities suitable for such medication include both naturally existing cavities and those formed by surgical procedures, e.g. small and confined body cavities such as the conjunctival cul-de-sac of the eye, surgical sites such as the interior of joints , and the interior of surgical wounds, particularly after surgery on delicate tissues such as in the eye. The microparticles used are unobtrusive, do not interfere with the surrounding tissues and are imperceptible to the patient. To administer a defined dose of medication, a predetermined number of microparticles containing a predetermined dose of a pharmaceutical compound are suspended in an external matrix of a material which dissolves relatively rapidly in the body fluids to make a dosage form of the invention which can be inserted as a unit into the body cavity which is to receive the medication. The unit dosage form is convenient for manipulation and provides a predetermined dose of the drug. Since the external matrix rapidly dissolves, the bulky dosage form does not remain in the cavity to distort the tissues and/or be perceived as uncomfortable by the patient. The microparticles can then distribute themselves throughout the cavity and, prior to their dissolution, release the drug in a controlled fashion.

Principal applications of the invention are the administration of medication to the eye by insertion of the dosage form into the conjunctival cul-de-sac, and to supply medication to a surgical site in the eye after performance of a filtration procedure for the relief of glaucoma which has become intractable to pharmaceutical treatment. In such a filtration procedure, a small flap of the sclera is lifted and a small incision is made into the eyeball to form a "bleb" which permits the escape of aqueous humor when the intraocular pressure becomes too high. The scleral flap is then sutured back in place. Occasionally, proliferation of fibroblasts within the surgical wound forms scar tissue which interferes with the drainage of aqueous humor through the bleb. It is desirable to place in the wound a pharmaceutical compound, such as an antimitotic compound, to inhibit the proliferation of fibroblasts. Another application is in arthroscopic surgery wherein it is sometimes desirable to provide antiinflammatory or antibacterial pharmaceuticals to the surgical site to aid in healing. The dosage of the invention is a premeasured drug for insertion, e.g. through the surgeon's arthroscope, directly into the surgical site. When the external matrix dissolves in the body fluids, the

microparticles are released, become distributed throughout the site, release their medication over on extended period, and, because of their small size and ultimate solubility in body fluids, will not interfere with the healing of the wound.

One embodiment of the ocular dosage form comprises a relatively thin sheet of a readily soluble matrix material having dimensions suitable for insertion into the upper or lower conjunctival cul-de-sac of the eye. Figure 1 shows a front elevational, somewhat diagrammatic view of a human eye, while Figure 2 shows a side elevation sectional view of the eye. An eyeball 102, having upper and lower eyelids 104 and 106, respectively, is covered for the greater part of its visible area by the sclera 108 and at its central portion by the cornea 110. The external surfaces of the eyeball and the facing surfaces of the eyelids are covered with an epithelial membrane, the conjuctiva 109. That portion of the conjunctiva 109 lining the eyelids is the palpebral conjunctiva while that portion covering the sclera 108 is the bulbar conjuctiva. The portion of the conjunctiva 109 which lines the upper eyelid 104 and the underlying portion of the bulbar conjuctiva define the upper sac 112 while that portion of the palpebral conjunctiva lining the lower eyelid 106 and the underlying portion of the bulbar conjunctiva form the lower sac 114. Upper and lower eyelashes are indicated at 116 and 118, respectively. The ocular insert 120 is in position in the lower sac 114 as it would be immediately after insertion and before the external matrix dissolves.

The ocular dosage form can be fabricated in any convenient shape for comfortable insertion into the sac of the eye, e.g. circular, elliptical, toroidal, bean-shaped, rod-shaped, banana-shaped, rectangular or lozenge-shaped. In cross-section it can be double convex, concavo-convex or rectangular. The actual shape is not significant, but it is preferred that the device be easy for the patient to handle and insert. The dosage forms shown in partial cross-section in Figures 3A, 3B and 3C illustrate some ocular shapes. Unlike known ocular insert dosage forms which are retained relatively intact for long periods and must be adapted for comfortable wear and preventing extrusion from the eye, the ocular insert dosage form of this invention rapidly dissolves in the tear fluids of the eye and need not be specially designed for comfortable wear and non-extrusion. It need only be retained in the sac of the eye with relative comfort for the short time required for the external matrix to dissolve in the tear fluid, i.e. from 10 seconds up to 20 or 30 minutes. The dimensions of the ocular dosage form of this invention are not critical with the lower limit determined by the amount of drug to be administered and the number of microcapsules it contains which are to be delivered for the desired pharmacological response and the need for a certain minimum size for convenient manipulation by the patient before being inserted into the eye. The upper limit is determined by the geometric limitations of the space within the eye. The dosage form must not be too large, so as to be inserted easily, and must be retained within the conjunctival sac until the matrix has dissolved.

The thickness of the dosage form may vary from 0.1 mm to 2.0 mm, preferably from 0.6 mm to 1.2 mm. Circular dosage forms may have diameters from 2 mm to 8 mm, preferably 5 to 6 mm. Generally elliptical dosage forms may have a width of 2 mm to 5 mm, preferably 3 to 4 mm, and a length of 5 mm to 10 mm, preferably 7 to 9 mm. Rectangular or ribbon-shaped dosage forms may have a width of 1.5 mm to 4 mm, preferably 2 to 3 mm, and a length of 5 mm to 12 mm, preferably 7 to 10 mm. Cylindrical dosage forms may have a diameter of 0.2 to 2 mm preferably 1 to 2 mm, and a length of 2 mm to 10 mm, preferably 7 to 9 mm.

Dispersed throughout the external matrix of the dosage form of the invention are a number of microcapsules which are controlled release reservoirs for a drug to be administered to the eye. Figures 3A, 3B and 3C illustrate typical dosage forms 120 of the invention, each partly cut away to show schematically a plurality of microparticle reservoirs 122 dispersed in a matrix 124 which is rapidly soluble in tear fluid. The microparticles are of a size to be retained in the eye after they are released by the dissolution of the external matrix and are made of a bioerodible material which will gradually be decomposed or dissolved in the tear fluid under the conditions prevailing in the eye. Eventually, the microparticles become small enough to be eliminated from the eye by being flushed through the punctum and down the tear duct by the continuous flow of tears, or may even be completely dissolved in the tears and thus removed from the eye. The dimension of the microparticles can range from 50 $\mu$m to 2 mm in diameter, such as 100 to 600 or 300 to 600 $\mu$m, e.g. 400 to 600 $\mu$m and can be of a generally spherical shape.

Figures 4, 5, 6 and 7 illustrate magnified views of sections of the dosage forms of the present invention showing microparticle drug reservoirs 122 dispersed in a rapidly soluble matrix 124. Some of the microparticles 122 are shown in partial section to illustrate their internal structure. Figure 4 shows microparticles 122 comprising solid particles 126, e.g. microcrystals of a drug, having a coating 128, e.g. of a synthetic or natural resin, which serves as the drug release-controlling element of the microparticle drug reservoir. With such encapsulated microparticles, controlled release is provided by diffusion of the drug through the coating 128, or the coating 128 may be permeable to water to dissolve the drug which then diffuses through the coating 128. Alternatively, the coating 128 may erode or dissolve in the tear fluid to

release the drug within.

Figure 5 illustrates an embodiment wherein the microparticles 122 comprise a liquid solution or suspension 130 of the drug encapsulated in a microcapsule wall 132 made from a natural or synthetic resin. Controlled release may be by diffusion of the drug through the microcapsule wall 132, by penetration of water from the tear fluid into the capsule wall to swell it or otherwise change its properties to permit diffusion of the drug through the wall 132, by osmotic rupture of the microcapsule due to diffusion of water through the capsule wall, by bioerosion of the capsule wall, or by any combination of these processes.

Figure 6 illustrates an embodiment of the invention wherein the microparticles 122 comprise a release control matrix 134 which is a solid material, e.g. gelatin, or a synthetic or natural resin, having dissolved or dispersed therein a drug 136 to be administered. The drug may be released from the microparticles by diffusion through the solid matrix 134, by penetration of the tear fluid into the matrix 134 to swell the matrix and mobilize the drug to diffuse out, or by bioerosion of the matrix 134 with release of the drug to the tear fluid. In microparticles of this type the drug 136 may be molecularly dispersed, i.e. dissolved, in the matrix 134 or may be dispersed as very fine particles throughout the matrix of the microparticle drug reservoirs. The drug release rate from the microparticle drug reservoirs of Figure 6 can be controlled by varying the material of the release control matrix. Different polymers having different rates of bioerosion in the ocular environment can be used. Alternatively, the properties of a release control matrix material can be modified to provide for a different release rate, e.g. gelatin as a release control matrix. Different populations of microparticle drug reservoirs can be prepared using gelatins of different degrees of hardening or cross-linking. These can be produced by exposing the microparticles to a hardening agent such as glutaraldehyde for varying lengths of time or to varying concentrations of a hardening agent to prepare particles having a release control matrix with a predetermined rate of drug release.

The microparticle drug reservoirs of Figure 6 can be further modified by being coated with another material to modify their release properties as in Figure 5. Multiple coatings can be used to adjust the time of release.

In all of the embodiments which employ microparticle drug reservoirs having a controlled release coating, the controlled release may be adjusted by varying the properties of the coating material. However, to provide a better control it is preferable to provide two or more populations of microparticle reservoirs, each population having a different controlled release property. For example, if a bioerodible coating is used on the microparticles, different populations of microparticles can have coatings which require different periods of time to bioerode and release the drug therein. The different bioerosion properties of the coatings may be provided in a number of ways, e.g. different populations of microparticles may have different numbers of coatings, coatings of different thicknesses, coatings of different materials, or coatings which have been treated differently to modify their bioerosion properties. Multiple coatings can be applied to the microparticles in the conventional manner, i.e. additional coating steps. Coatings of greater thickness may be applied by lengthening the coating application process or otherwise modifying the coating process to produce a coating of greater thickness. Two or more populations of microparticle drug reservoirs can be coated with different materials, e.g. two different natural or synthetic resins which have different rates of bioerosion in the environment of the eye. The coatings can also have their properties altered by treatments after the coating step, e.g. microparticles coated with gelatin may have their coatings treated with a cross-linking agent, e.g. formaldehyde or glutaraldehyde, for a period of time to harden the coating and slow the rate of bioerosion. Treatments with different concentrations of cross-linking agents or for different periods of time can be used for coatings which bioerode at different rates. The use of different coatings will cause the drug to be released from the different populations of reservoirs at different times.

The microparticle drug reservoirs of this invention can provide for successive administration of different drugs by incorporating the drugs into separate populations of reservoirs, each population being designed to release its drug at a different time. The method can be extended to administration of drugs alternately in succession, using only a single dosage form of the invention.

Figure 7 illustrates an embodiment wherein the microparticles are in the form of thin disks 138 of a release control matrix having a drug dissolved or dispersed as very fine particles in the disks. The drug release mechanism is essentially the same as for the particles shown in Figure 6. Since the flat surface of the disks is very large compared to the surface of the edge of the disk, the surface tends to remain practically the same as the particle erodes and the rate of drug release thereby tends to be constant (zero order).

In another embodiment the drug is encapsulated in a liposome to achieve the advantages inherent in the use of liposome drug vehicles. The liposomes are dispersed in an external rapidly soluble matrix to provide a dosage form according to the invention.

In another embodiment, one drug is incorporated into the external matrix for rapid dissolution and drug

release, while a second drug, intended for longer duration therapy, is incorporated into the microcapsules and slowly released therefrom. Alternatively, the same drug may be incorporated into both the outer matrix, to provide an immediate surge of drug, and the microcapsules, to provide for continuous delivery.

The number of microparticles contained in the ocular insert will vary depending on the amount of pharmaceutical to be administered, the microcapsule size, the amount of pharmaceutical compound contained in each microcapsule and the like.

The external matrix material used in the controlled release dosage composition of the invention may be any pharmaceutically acceptable solid material which is sufficiently cohesive to retain the microcapsules within it and which dissolves rapidly in body fluids. In an ocular dosage form, the external matrix should have sufficient mechanical strength to withstand the handling needed for commercial manufacture and distribution as well as for insertion into the eye. It should be rapidly soluble in tear fluid after it has been inserted into the eye. Conveniently, the external matrix should retain its strength and shape only long enough for it to be inserted into the cul-de-sac of the eye, and then should dissolve. It is preferable for the external matrix to dissolve in a period of 1 to 2 minutes, although shorter or longer periods for dissolution are not excluded. Periods of up to 30 minutes before dissolution is complete are acceptable, although for patients with normal tear volume, there is little reason for delaying the dissolution of the external matrix for periods longer than several minutes.

Suitable materials for the external matrix include, but are not limited to-sodium glycerolate; glycolic acid; lactic acid; dextrose; sodium acetate; potassium acetate; gelatin; dextran; sorbinose; sorbitol; mannitol; glucose 6-phosphate, dipotassium salt; calcium levulinate; sodium ascorbate; sodium gluconate; sodium glycerophosphate; sodium salicylate; sodium succinate; and sodium tartrate. Particularly preferred are polyvinyl alcohol, high molecular weight polymers of ethylene oxide, mannitol, and hydroxypropyl cellulose. These materials may be used in lyophilized form by mixing the microparticles with a measured amount of the matrix material and preparing a dosage form by direct compression or extrusion to form a coherent mass. Alternatively, the microparticles may be suspended in a solution of one of these materials, the solvent for the material being a non-solvent for the microparticles, and the resulting suspension cast into a film by evaporation, with the dosage form then being punched, cut, or stamped out of the film. Shapes for the microparticle-containing external matrix, in the final form, include spheres; circular, rectangular, or elliptical films or discs; or rods. The shape of any specific system is determined by its intended use. Thus either circular, elliptical, or rod-shaped systems may be inserted in the cul-de-sac of the eye to release drugs of value in treating ocular diseases; pellet or rod-shaped systems may be conveniently placed in the cavity following arthroscopic surgery; or a rectangular film may be placed in the sclera under the conjunctival flap at the close of a filtering procedure in the surgical treatment of glaucoma. These systems may range in sizes appropriate to the intended use. Sizes for use in the cul-de-sac of the eye are disclosed above. Systems for the glaucoma surgery procedure are rectangular films that may range from 4 to 12 millimeters square, with a preferred size of 8 millimeters square; with a thickness that may range from 0.2 millimeters to 2.0 millimeters wish a preferred thickness of about 0.6 millimeters. Systems for the arthroscopic or other surgical cavities are optimally represented by rods 1 mm in diameter and 10 mm in length, or by spherical pellets 2 mm in diameter, although the rods may range from 0.5 mm to 4 mm in diameter and from 4 to 20 mm in length, and the spherical pellets may range from 1 to 5 mm in diameter. Multiple systems may be employed for placement in surgical cavities to provide for a range of dosages.

The microparticle controlled release drug reservoirs for ocular dosage forms comprise a predetermined amount of a drug dispersed in a matrix or provided with a coating which provides for gradual release of the drug to a surrounding liquid medium. The drug release rate controlling material used in the microparticle drug reservoirs of this invention may be any non-toxic bioerodible material, inert toward the drug to be encapsulated, which can form a particle containing a physiologically active drug and maintain its integrity in the tear fluid environment for a period of time to provide an extended release of the drug. The pharmaceutical compound is only released into the ocular environment when the drug diffuses through the rate controlling matrix or when the release rate controlling material is slowly eroded. As used in this application the term "drug release rate controlling material" is intended to include only those materials which truly function for controlled release of a drug over a period of some time. Fillers, binders and colorants are not included among such materials. The drug release rate controlling material must be bioerodible, i.e. it must innocuously disintegrate or break down from a unit structure or enclosure over a prolonged period of time in response to the environment of the eye by one or more physical or chemical degradative processes, e.g. enzymatic action, hydrolysis, ion exchange or dissolution by solubilization, emulsion formation or micelle formation. The term "bioerode" is defined as the process by which such disintegration takes place. Such bioerosion serves two purposes. The drug is released into the ocular environment and, on the other hand, the microparticles are reduced in size so that they do not remain

indefinitely within the eye but are either totally dissolved or reduced to a size at which they are eliminated by flushing through the punctum by the flow of tear fluid.

The materials used to form the microparticles are bioerodible and non-toxic substances which are compatible with the drug to be administered and which are capable of forming a film surrounding or enclosing a drug particle or a matrix within which a drug is dispersed either in particulate form or as a molecular dispersion. One example of a biocompatible, bioerodible polymeric material is C101/ct from ALZA Corporation of Palo Alto, Ca which is described by R. C. Capossa et al. in "Polymeric Delivery Systems", Midlano Macromolecular Monographs, New York, Gordon & Breach at pp 59-73 (1978). Polymer C101/ct is hydrophobic and undergoes a surface chemical reaction with the surrounding aqueous medium which results in erosion and concomitant release of drug such that no residue remains after total drug depletion.

For sparingly soluble drugs, materials can be used which form water-swollen gels, such as gelatin, hydroxypropyl cellulose, or polyoxyethylene-polyoxypropylene block copolymers. These gels swell when introduced into the aqueous environment of the eye, but retain the drug within the swollen gel. The drug then diffuses from the interior of the swollen gel to the tear fluid and thereby contacts the ocular tissues. The release rate is largely governed by the rate of dissolution of the drug, and for some poorly soluble drugs this presents a clinically useful release pattern.

For water-soluble drugs, which would be released too rapidly from water-swollen gels and washed away by the continuous flow of tears, alternative materials may be used. Such drugs can be incorporated into a matrix material that is not highly hydrophilic. Examples of such materials are poly(glycolic acid); poly(lactic acid); poly(hydroxybutyric acid)j aliphatic polyesters, such as polycaprolactone; polyacryl starch; polyanhydrides; poly(ortho esters), with or without latentiated acid catalyst; poly(DL-lactamide); copolymers of leucine and glutamic acid, or lactic and aminocaproic acid; and glycosaminoglycans. From such materials the soluble drug is not readily leached out and the release rate depends on a combination of factors, including diffusion of the drug through the matrix, leaching and surface erosion. Ultimately such matrices totally dissolve. A particular polymer class for use in the invention is the poly(ortho ester) class, e.g. as described in US-A-4,180,646. Such polymers are typically of the following formula:

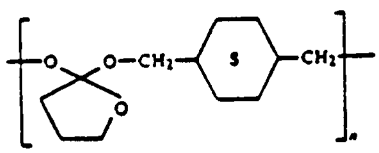

where n is 10 to 1000. Other disclosures of poly(ortho ester) polymers include US-A-4,066,747; US-A-4,070,347; US-A-4,079,038; US-A-4,093,709; US-A-4,115,544; US-A-4,119,579; US-A-4,131,648; US-A-4,136,252; US-A-4,138,344; US-A-4,155,992; US-A-4,186,185; US-A-4,246,397; and US-A-4,282,201.

For example, an ophthalmic drug can be incorporated into a matrix material from which it is released by enzymatic degradation of the matrix. The enzymes to perform this degradation may be present in the tears of the patient, e.g. lysozyme, or one or more enzymes may be added to the matrix itself. Matrix materials of this type include chitin, n-acetylglucosamine, de-acetylated chitin, and cyclodextrin.

The controlled release matrix materials also include polysaccharides, e.g. dextran, starch, or the like, and copolymers of lactic acid and glycolic acid. These materials may be used for an inner core of a microsphere containing the drug, and a capsule wall of the same material may envelop the core, if desired to adjust the rate of drug release. These materials release the drug by erosion, and the particles coated with a capsule wall which contains no drug do not at first contribute to the medication. After the microcapsule wall has eroded, however, the originally coated microcapsules begin to release their drug at a time when uncoated capsules may be exhausting their supply. This permits a mixture of coated and uncoated particles of this type to provide for an extended release of the drug.

The drug may be incorporated into microparticles of complexes such as a starch-borate complex or a polyvinyl alcohol-borate complex.

Another reservoir incorporates a drug into a polymeric matrix, e.g. a polypeptide such as gelatin, a polysaccharide such as sodium alginate or polyvinyl alcohol, which is then formed into microparticles followed by partial polymerization and/or cross-linking of the matrix material by exposure to suitable reagents. Such reagents are well known and include reagents such as an aldehyde (e.g. glutaraldehyde) for gelatin, calcium chloride for sodium alginate, and borate for polyvinyl alcohol. Depending on the conditions of manufacture, the microparticles may be hardened throughout or only a surface layer of predetermined thickness may be hardened. These reservoirs release the drug at a controlled rate by diffusion through the

polymerized matrix or through the barrier formed by the polymerized and/or cross-linked surface, and the particles themselves ultimately dissolve and are eliminated.

When the drug is dissolved or dispersed in a hydrolytically stable, water-soluble polymer, such as gelatin, which has been partially insolubilized by covalent cross-linking, e.g. by treatment with formaldehyde, glutaraldehyde or another cross-linking agent, the matrix material will swell in the tears of the eye, producing a hydrogel, from which the drug, in particular a water-insoluble drug, will diffuse out, rapidly at first but more slowly with the passage of time. At the same time the matrix will decompose, increasing the bioavailability of the drug. By adjusting the composition and amount of cross-linking of the polymer, the decrease in diffusion and the enhanced bioavailability due to decomposition of the matrix can be made to compensate each other to produce an approximately zero order rate of drug delivery.

Another hardened protein drug reservoir is prepared by incorporating the drug into microparticles of a gelled protein solution, e.g. a gelled albumin/water solution. Such particles can be prepared by dissolving the drug in an aqueous albumin solution, adding the solution dropwise to an immiscible organic phase, e.g. an oil in which the drug is not soluble, with slow constant stirring, the rate of the stirring being adjusted to maintain optimum size of the albumin/water spheres. The spheres are then hardened by adding to the mixture a suitable reagent, e.g. 2,3-butadione or glutaraldehyde solution. The hardened spheres may then be filtered and washed with a solvent which removes the oil from their surfaces. Alternatively, the reservoirs can be produced by injection molding by mixing the solid drug particles, the polymer, such as gelatin, and the cross-linking agent followed by injecting and molding the mixture into microspheres.

Microparticles comprising a drug incorporated into degradable starch spheres are prepared by emulsion polymerization of a soluble potato starch hydrolysate. Amylase may be incorporated into such spheres as an erosion enhancer.

Gelled alginate matrix microparticles may be prepared by adding a soluble drug, or a suspension of a sparingly soluble drug, to a saline solution of sodium alginate, then adding this solution or suspension dropwise to a solution of calcium chloride, which cross-links and gels the alginate. The solidified spheres are then washed and treated with a polyamino acid in a saline buffer to harden the surface. The alginate inside the spheres can then be liquified by sequestering the cross-linking calcium with citrate by washing the spheres with an aqueous solution of sodium citrate. Such spheres release the drug by diffusion through the thin surface membrane and the spheres are ultimately eroded and eliminated from the eye.

These matrix materials are illustrative only. Any bioerodible material which is compatible with the drug, non-toxic, non-irritating, and has the desired erosion and/or diffusion rate properties can be used. The preferred materials are the polyesters, alginates, gelatin, cross-linked poly(peptides) and poly(ortho esters).

Ophthalmic drugs for the invention include anti-inflammatory corticosteroids, such as fluorometholone, dexamethasone, prednisolone, dexamethasone 21-phosphate, fluocinolone, medrysone, methylprednisolone, prednisolone 21-phosphate, prednisolone acetate, betamethasone and triamcinolone; carbonic anhydrase inhibitors, such as ethoxzolamide, methoxzolamide, acetazolamide, trifluoromethazolamide and derivatives thereof, topiramate and (1,2,3,4-tetrahydro-2-naphthalenyl)methylsulfamic acid, both as described in US-A-4,513,006; beta-adrenergic blocking agents, such as metipranolol, bevantolol, timolol and metoprolol; combinations of a beta-blocking agent with pilocarpine, such as metipranolol/pilocarpine, bevantolol/pilocarpine, and timolol/pilocarpine; antibiotics, such as a member of the aminoglycoside family, e.g. gentamycin, or a member of the quinolone family, such as norfloxacin, or a member of the cephalosporin family, or neomycin, polymyxin, tetracycline, chlortetracycline, bacitracin, gramicidin, oxytetracycline, chloramphenicol, penicillin, vancomycin or erythromycin; a steroid-antibiotic combination; miotics and anticholinesterases such as pilocarpine, eserine salicylate, carbachol, diisopropylfluorophosphate, phospholine iodide, and demecarium bromide; antiviral agents, such as idoxuridine, trifluorothymidine or adenine arabinoside; sympathomimetics, such as epinephrine; combinations such as guanethidine/epinephrine, guanadrel/epinephrine, guanethidine/terbutaline, or guanadrel/terbutaline; alpha-adrenergic blocking agents, such as thymoxamine; diuretics, such as triamterene; antimitotics, such as 5-fluorouracil; aldose reductase inhibitors, such as sorbinil, tolrestat, or statil; mast cell stabilizers, such as sodium cromoglycate; agents for treating dry eye syndrome, such as vitamin A (retinol) or vitamin A acid (retinoic acid) in either the cis or trans form, or sodium chloride; agents to enhance wound healing, such as epidermal growth factor (EGF) or fibronectin; immunosuppressive agents, such as cyclosporin A; non-steroidal anti-inflammatory agents, such as phospholipase inhibitors, indomethacin, aspirin, pyrazolac, ketorolac, diclofenac or panaprophen; alpha-adrenergic agonists, such as clonidine; antibacterials such as sulfonamides, sulfacetamide, sulfamethizole, sulfisoxazole, nitrofurazone and sodium propionate; antiallergenics such as antazoline, methapyryline, chlorpheniramine, pyrilamine and prophenpyridamine; decongestants such as phenylephrine, naphazoline, and tetrahydrazoline; and mydriatics such as atropine (e.g. sulfate), cyclopentolate, homatropine, scopolamine, tropicamide, eucatropine, and hydroxyamphetamine.

In another embodiment for surgical implantation, drugs include antimitotic agents such as adenosine arabinoside or 5-fluorourocil for use during a filtering procedure for the surgical treatment of glaucoma; antibiotics, as above; and anti-inflammatory agents, as above, for use during glaucoma or arthroscopic surgery.

After the preparation of the microparticle drug reservoirs, a quantity is incorporated into an external matrix material and the final dosage form, for example an ocular insert, is formed from the mixture. In this ocular usage, in order to provide a relatively extended period of medication to the eye, it is preferable to incorporate two or more different populations of microparticles, each population having different drug release characteristics due to variations in the size, surface, hardening, coating or drug concentration of the microparticles. The microparticles are suspended into an aqueous solution of a suitable water-soluble colloid, such as polyvinyl alcohol, a high molecular weight polymer of ethylene oxide, or hydroxypropyl cellulose. After thorough mixing, a film is cast and dried. Dosage forms of the desired size are cut or stamped from the film. Alternatively, the microparticles can be mixed with the external matrix material in dry or lyophilized form, extruded under pressure to form a thin cylinder and cut into lengths appropriate for dosage forms. A third procedure comprises mixing the microparticles with the external matrix material in dry form and molding dosage forms by compression molding.

Following the formation of the dosage forms, they are packaged individually and sterilized either by exposure of the closed package to ethylene oxide gas by conventional procedures or by exposure to ionizing radiation. In a final packaging step the individual dosage form packages are enclosed in a suitable moisture-barrier package for storage and distribution.

While the external matrix of the ocular dosage form will ordinarily be readily soluble in the tear fluid of the patient, if the patient also suffers from a deficiency of naturally produced tear fluid, a conventional artificial tear fluid can be added to the eye immediately after the dosage form has been inserted to promote dissolution of the external matrix and the dispersion of the microparticles.

The amount of drug in the dosage form will vary widely depending on the drug and the body site for administration. Selection of a particular dose in a given situation will be readily apparent to the skilled practitioner. For ocular drugs the release rate (dose) of the drug will vary from 10 µg/h to 50 µg/h in most cases. For common anti-glaucoma drugs such as pilocarpine the preferred rate is about 40 µg/h and a preferred rate for the antimitotic drug administration after a filtration procedure would be about 10 µg/h.

The total amount of drug to be incorporated into the dosage form is then easily calculated from the release rate and the period of administration. For example, for treatment of glaucoma by weekly administration, the dosage form should preferably contain about 7 mg of pilocarpine. Many adjustments can be made in the amount of drug contained in a particular dosage form to adapt it for treatment of various conditions and various individual patients.

The invention will now be illustrated by the following illustrative examples.

Example 1

100 ml of a 5 % gelatin solution (Sigma Chemicals, 225 bloom) is mixed with 2 g of a finely-divided drug which is only slightly (sparingly) soluble in water in a container immersed in a water bath kept at 40 ° C. The mixture is stirred at 200-300 rpm with a propeller type stirrer coated with polytetrafluoroethylene during the entire preparation procedure. When thee drug is completely mixed into the gelatin solution, 100 ml of acetone are added to the suspension over a period of 10-15 minutes. Under these conditions microcapsules spontaneously form. When the formation of the capsules is complete, about 50 minutes after the addition of acetone, the water bath used to maintain the temperature of the mixture at 40 ° C is removed and exchanged for an ice bath. When the temperature of the mixture has dropped to about 5 ° C, 2 ml of an aqueous 50 % glutaraldehyde solution are added dropwise and the ice bath is then removed. Depending on the desired degree of cross-linking, one to four hours after glutaraldehyde is added the capsules are filtered by vacuum and washed with acetone several times (3-4 washes). This procedure provides microparticles consisting of drug totally encapsulated by cross-linked gelatin, and is useful with drugs that have very low solubility.

Example 2

100 mg of the sparingly soluble drug and 200 mg of albumin are added to 0.8 ml of a pH 7 buffer solution. One milligram of sodium dodecylsulfate and 0.2 ml of chilled glutaraldehyde are also added. After mixing this mixture is added, with rapid stirring, to 100 ml of a 1:4 mixture of corn oil/petroleum ether. Stirring is continued at room temperature for 1 hour. The supernatant liquid is decanted and discarded, and

the microcapsules are then washed with petroleum ether or alcohol, dried and stored. This procedure produces microcapsules of from 100 to 200 µm in size.

Example 3

A weighed amount of a drug which is only slightly soluble in water is added to a sufficient quantity of chloroform to effect dissolution. Glutaraldehyde is added to reach a final concentration of 0.1%. This mixture is then added, while mixing, to an equal volume of an aqueous solution containing 5% gelatin and 40% methanol. The microcapsules form during the mixing operation, and are subsequently separated and washed to remove the chloroform.

It will be understood that microcapsules made in Examples 1 to 3 are formed using various glutaraldehyde exposures (varying both concentration and exposure time), producing, in any one procedure, different batches of microcapsules from each batch. Combining these and incorporation of this mixture into the outer matrix results in a device whose release rate profile reflects the net composite of the contributing microcapsules.

Example 4

A 30% disperson of microfine drug particles is formed in a 10% gelatin aqueous solution at elevated temperature (30° C to 40° C). The warm suspension is cast into a film and chilled to gel the suspension. Following the gelling step, the film is immersed in a 1% aqueous glutaradehyde solution for from 0.5 to 3 hours depending on the desired degree of cross-linking. The hardened film is cut into small pieces (area from 0.5 to 1.0 $cm^2$). These pieces are then ground to 100-200 µm size using a suitable high-speed blender.

Example 5

A quantity of 2.5 g of a water soluble drug plus 10 g of polylactic acid are added to a sufficient quantity of methylene chloride to effect solution. This solution is added slowly with stirring to a 5% solution of polyvinyl alcohol in water. The stirring rate is slowly increased to 2000 rpm. A vacuum is applied to reduce the methylene chloride concentration to approximately half volume. The suspension is then centrifuged and decanted, with the remaining microcapsules dispersed in water. Evaporation is continued until the methylene chloride is completely removed, to yield a suspension of the microparticles in water.

Example 6

The procedure of Example 5 is followed, except that poly(beta-hydroxy-butyrate) is substituted for the polylactic acid, and the methylene chloride is replaced with chloroform.

Example 7

The drug to be delivered is added to normal saline to form a solution that may range in concentration from 10% up to a saturated solution with respect to the drug. Alternatively, the drug may be added in excess of its solubility so that it is present as a suspension in saline. Sodium alginate is also added to this solution (or suspension) to the extent of from 1% to 3% sodium alginate. This solution (suspension) is then added dropwise, using a micropipette or other small orifice, into an aqueous solution of from 1% to 3% calcium chloride. The microspheres that then form as a consequence of the hardening by calcium ion of the alginate are washed and then treated with a solution of from 0.001% to 0.10%, preferably 0.125% poly-L-lysine, or other polyamino acid, in saline, buffered to pH 7.0. The microcapsules are then treated with an aqueous solution containing from 0.01% to 0.10% sodium citrate solution, to remove calcium from their interiors, washed and dried.

Example 8

An aqueous solution is made with two ingredients: one being the drug to be delivered, in from 20% concentration up to a saturated solution; and the other being a biologically inert carbohydrate material, such as lactose, mannitol, dextran, dextrin, or glucose. This solution is added to a like volume of a non-aqueous medium, such as vegetable oil or hexane, with sufficient mixing to form a pre-emulsion. This pre-emulsion

is added slowly with rapid blending to an acetone solution containing 0.1% to 0.2% polysorb 80, after which the carbohydrate precipitates in the form of microspheres containing the drug. The microspheres are separated, washed with additional acetone-polysorb 80 solution and dried.

Example 9

An aqueous solution of polyvinyl alcohol is prepared having a concentration of 20% to 40% by weight of polyvinyl alcohol. Microparticles from any one of Examples 1-8 are added to form a suspension of the microparticles having a particle concentration of from 20% to 76% by weight. The suspension is cast into a thin film and air dried, after which the dosage form is cut from the hardened film by a stamping or cutting operation.

Example 10

Dry microparticles containing encapsulated drugs prepared by any one of Examples 1-8 are mixed with a dry powder of an ethylene oxide polymer, and extruded under pressure to form a thin, cylindrical, solid extrusion, which is then cut into individual dosage forms. The final cylindrical devices may vary from 0.2 mm to 2 mm in diameter, and from 2 mm to 10 mm in length, with a preferred diameter of 1 to 2 mm and a preferred length of 7 to 9 mm.

Example 11

The dry mixture prepared as in Example 10 above was molded by compression molding to form a dry device having the desired shape, e.g. circular, elliptical, rod-shaped or ribbon-shaped.

Example 12

Following Example 5 above, microparticles of pilocarpine hydrochloride dispersed in a release rate control matrix of poly(lactic acid) were prepared. The microparticles were incorporated into an external matrix of polyvinyl alcohol by the procedure of Example 9, and a film having a dried thickness of about 1.0 mm was cast from the mixture. Anti-glaucoma dosage forms having a rectangular shape 3 mm wide and 10 mm long were cut from the polyvinyl alcohol film. When the dosage form was immersed in 0.9% saline solution to simulate natural tears, the external matrix dissolved within a few seconds yielding a suspension of the microparticle drug reservoirs in the aqueous solution. The microparticles were observed periodically over several hours to gradually become smaller in size releasing the pilocarpine to the solution and eventually to dissolve completely.

Example 13

Microparticles of cross-linked gelatin containing the sparingly soluble steroid antiinflammatory drug fluorometholone were prepared according to Example 1. The microparticles were then incorporated into thin lamellar ocular dosage forms having an external matrix of polyvinyl alcohol by the procedure of Example 9. When the dosage forms were placed into the cul-de-sac of the eye of a rabbit, the external matrix was observed to dissolve completely within fifteen minutes.

The rate of release of the drug was studied by immersing a dosage form in a simulated tear fluid of physiological saline. Immediately upon immersion, the external matrix dissolved, releasing the microcapsules into the surrounding saline solution. The amount of fluorometholone in the solution was monitored spectrophotometrically over several days. A plot of the amount of drug released versus time is illustrated in Fig. 8. Each point represents the average of three measurements and the standard deviation is shown by a vertical line through each point.

Example 14

Microparticles of cross-linked albumin containing fluorometholone were prepared according to the procedure of Example 2. The microparticles so prepared were then incorporated into polyvinyl alcohol dosage forms as in Example 9. Dosage forms were prepared using two different batches of cross-linked albumin microparticles. When the dosage forms were placed into the cul-de-sac of the eye of an experimental rabbit, the external matrix dissolved completely within fifteen minutes. The rate of release of

the drug was studied by immersion in a simulated tear fluid of physiological saline. Immediately upon immersion, the external matrix dissolved, releasing the microcapsules. The amount of fluorometholone in the solution was monitored spectrophotometrically over several days. A plot of the amount of drug released versus time for each batch of microcapsules is illustrated in Fig. 9.

## Claims

1. A composition for controlled release of a drug contained therein to a body fluid, said composition comprising an external matrix soluble in said body fluid, and microparticles dispersed in said external matrix, characterised in that: said microparticles comprise a drug dispersed within a bioerodible release rate controlling matrix and are capable of releasing the drug gradually to said body fluid; and the external matrix is substantially completely soluble in the body fluid within from 10 seconds to 30 minutes.

2. The composition of claim 1, which comprises an ocular dosage form for controlled release of the drug to the tear fluid of a mammalian eye, wherein said external matrix is rapidly soluble in said tear fluid of the eye, said microparticles are capable of releasing the drug gradually to said tear fluid, and said microparticles are of a size to be retained in the eye for a period of time long enough to provide an effective dose of the drug over an extended time period to the eye.

3. The composition of claim 2, wherein said microparticles have a dimension ranging from 50 µm to 2 mm in diameter.

4. The composition of claim 3, wherein said release rate controlling matrix is poly(lactic acid), poly(beta-hydroxybutyrate), cross-linked alginate, cross-linked gelatin, or a bioerodible carbohydrate.

5. The composition of claim 4, wherein said release rate controlling matrix is a poly(ortho ester).

6. The composition of claim 5, wherein said poly(ortho ester) is of the following formula:

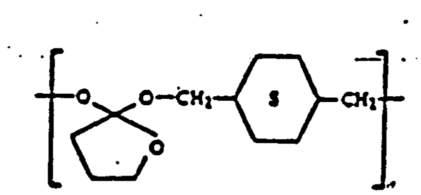

wherein n is the degree of polymerisation and is from 10 to 1000.

7. The composition of any one of claims 1 to 6, wherein said drug is selected from the group consisting of anti-inflammatory agents; carbonic anhydrase inhibitors; beta-adrenergic blocking agents; antiglaucoma agents, and combinations of any of the foregoing; antibiotics; or combinations of anti-inflammatory and antibiotic agents; antiviral agents; combinations of anti-glaucoma and alpha-adrenergic blocking agents; diuretics; aldosereductase inhibitors; mast cell stabilisers; agents to enhance wound healing; immunosuppressive agents; non-steriodal anti-inflammatory agents; alpha andrenergic agonists; and mydriatics.

8. The composition of claim 7, wherein said pharmaceutical compound is fluorometholone, acetazolamide, metipranolol, pilocarpine, epinephrine, vancomycin, penicillin, chloramphenicol, thymoxamine, triamterene, sorbinil, sodium cromoglycate, fibronectin, epidermal growth factor, cyclosporin A, indomethacin, ketorolac, clonidine, or atropine.

9. A process for the production of a composition of any one of claims 1 to 8, comprising incorporating said drug into microparticles of a controlled release composition and suspending said microparticles in a solid matrix which is readily soluble in said body fluid.

## Revendications

**1.** Composition pour la libération contrôlée d'un médicament qui y est contenu dans un liquide de l'organisme, ladite composition comprenant une matrice externe soluble dans ledit liquide de l'organisme et des microparticules dispersées dans ladite matrice externe, caractérisée en ce que : lesdites microparticules comprennent un médicament qui est dispersé dans une matrice bioérodable contrôlant la vitesse de libération et peuvent libérer graduellement le médicament dans ledit liquide de l'organisme ; et ladite matrice externe est sensiblement complètement soluble dans le liquide de l'organisme en 10 secondes à 30 minutes.

**2.** Composition selon la revendication 1, qui comprend une forme d'administration oculaire pour la libération contrôlée du médicament dans le liquide lacrymal de l'oeil d'un mammifère, dans laquelle ladite matrice externe est rapidement soluble dans ledit liquide lacrymal de l'oeil, lesdites microparticules peuvent libérer graduellement le médicament dans ledit liquide lacrymal et lesdites microparticules ont une taille telle qu'elles sont maintenues dans l'oeil pendant une période suffisamment longue pour fournir à l'oeil une dose efficace du médicament pendant une période prolongée.

**3.** Composition selon la revendication 2, dans laquelle lesdites microparticules ont une dimension comprise entre 50 µm et 2 mm de diamètre.

**4.** Composition selon la revendication 3, dans laquelle ladite matrice contrôlant la vitesse de libération est faite de poly(acide lactique), de poly(bêta-hydroxybutyrate), d'alginate réticulé, de gélatine réticulée ou d'un glucide bioérodable.

**5.** Composition selon la revendication 4, dans laquelle la matrice contrôlant la vitesse de libération est un poly(ortho-ester).

**6.** Composition selon la revendication 5, dans laquelle ledit poly(ortho-ester) répond à la formule suivante :

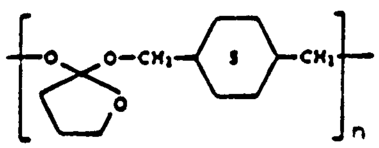

dans laquelle n est le degré de polymérisation et vaut de 10 à 1 000.

**7.** Composition selon l'une quelconque des revendications 1 à 6, dans laquelle ledit médicament est choisi dans le groupe constitué par les agents anti-inflammatoires ; les inhibiteurs de l'anhydrase carbonique ; les agents bêta-bloquants adrénergiques ; les agents antiglaucomateux et les associations quelconques des précédents ; les antibiotiques ; ou les associations d'agents anti-inflammatoires et antibiotiques ; les agents antiviraux ; les associations d'agents antiglaucomateux et d'agents alpha-bloquants adrénergiques ; les diurétiques ; les inhibiteurs de l'aldose réductase ; les stabilisants des mastocytes ; les agents facilitant la cicatrisation des plaies ; les agents immuno-suppresseurs ; les agents anti-inflammatoires non stéroïdiens ; les agonistes alpha-adrénergiques ; et les mydriatiques.

**8.** Composition selon la revendication 7, dans laquelle ledit composé pharmaceutique est la fluorométholone, l'acétazolamide, le métipranolol, la pilocarpine, l'épinéphrine, la vancomycine, la pénicilline, le chloramphénicol, la thymoxamine, le triamtérène, le sorbinil, le cromoglycate de sodium, la fibronectine, le facteur de croissance épidermique, la ciclosporine A, l'indométhacine, le kétorolac, la clonidine ou l'atropine.

**9.** Procédé pour la préparation d'une composition selon l'une quelconque des revendications 1 à 8 comprenant l'incorporation dudit médicament à des microparticules d'une composition assurant la libération contrôlée et la mise en suspension desdites microparticules dans ladite matrice solide qui est facilement soluble dans ledit liquide de l'organisme.

## Patentansprüche

1. Zusamensetzung zur gesteuerten Freisetzung eines darin enthaltenen Arzneiwirkstoffes an eine Körperflüssigkeit, welche Zusammensetzung eine in dieser Körperflüssigkeit lösliche äußere Matrix und in dieser äußeren Matrix dispergierte Mikroteilchen umfaßt, dadurch gekennzeichnet, daß diese Mikroteilchen einen in einer biologisch erodierbaren, die Freisetzungsgeschwindigkeit steuernden Matrix dispergierten Arzneiwirkstoff umfassen und zur allmählichen Freisetzung des Wirkstoffes in die Körperflüssigkeit befähigt sind und daß die äußere Matrix in der Körperflüssigkeit innerhalb von 10 Sekunden bis 30 Minuten im wesentlichen vollständig löslich ist.

2. Zusammensetzung nach Anspruch 1, welche eine Augendosierungsform für die gesteuerte Freisetzung eines Arzneiwirkstoffes an die Tränenflüssigkeit des Auges eines Säugetieres umfaßt, worin die äußere Matrix in der Tränenflüssigkeit des Auges rasch löslich ist und die Mikroteilchen zur allmählichen Freisetzung des Arzneiwirkstoffes an die Tränenflüssigkeit befähigt sind, und die Mikroteilchen eine solche Größe aufweisen, daß sie während einer ausreichend langen Zeitdauer im Auge verbleiben, um eine wirksame Dosis des Arzneiwirkstoffes während eines ausgedehnten Zeitraumes dem Auge zur Verfügung zu stellen.

3. Zusammensetzung nach Anspruch 2, worin die Mikroteilchen eine Größe im Bereich von 50 µm bis 2 mm im Durchmesser aufweisen.

4. Zusammensetzung nach Anspruch 3, worin die die Freisetzungsgeschwindigkeit steuernde Matrix aus Poly(milchsäure), Poly(beta-hydroxybutyrat), vernetztem Alginat, vernetzter Gelatine oder einem biologisch erodierbaren Kohlenhydrat besteht.

5. Zusammensetzung nach Anspruch 4, worin die die Freisetzungsgeschwindigkeit steuernde Matrix ein Poly(orthoester) ist.

6. Zusammensetzung nach Anspruch 5, worin der Poly(orthoester) die nachstehende Formel aufweist:

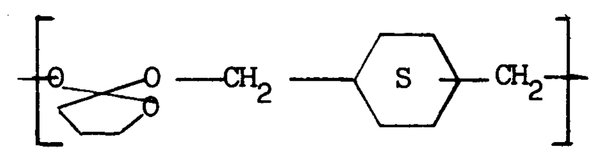 ,

worin n den Polymerisationsgrad darstellt und einen Wert von 10 bis 1.000 aufweist.

7. Zusammensetzung nach einem Ansprüche 1 bis 6, worin der Arzneiwirkstoff aus der aus entzündungshemmenden Mitteln; Carboanhydrase-Inhibitoren; Beta-Rezeptorenblockern; Antiglaucommitteln und beliebigen Kombinationen der vorstehenden Mitteln; Antibiotika; oder Kombinationen von entzündungshemmenden und antibiotischen Mitteln; antiviralen Mitteln; Kombinationen von Antiglaucommitteln und Alpha-Rezeptorenblockern; Diuretica; Aldosereductaseeinnibitoren; Mastzellen-Stabilisatoren; die Wundheilung fördernden Mitteln; Immunsuppressiva; nicht-steroiden entzündungshemmenden Mitteln; Alpha-Adrenergenagonisten; und Mydriatica bestehenden Gruppe ausgewählt ist.

8. Zusammensetzung nach Anspruch 7, worin die pharmazeutische Verbindung Fluormetholon, Acetazolamid, Metipranolol, Pilocarpin, Epinephrin, Vancomycin, Penicillin, Chloramphenicol, Thymoxamin, Triamteren, Sorbinil, Natriumcromoglycat, Fibronectin, Epidermis-Wachstumfaktor, Cyclosporin A, Indomethacin, Ketorolac, Clonidin oder Atropin ist.

9. Verfahren zur Herstellung einer Zusammensetzung nach einem der Ansprüche 1 bis 8, umfassend das Einbringen des Arzneiwirkstoffes in Mikroteilchen einer Zusammensetzung mit gesteuerter Freisetzung und ein Suspendieren dieser Mikroteilchen in einer festen Matrix, die in der Körperflüssigkeit leicht löslich ist.

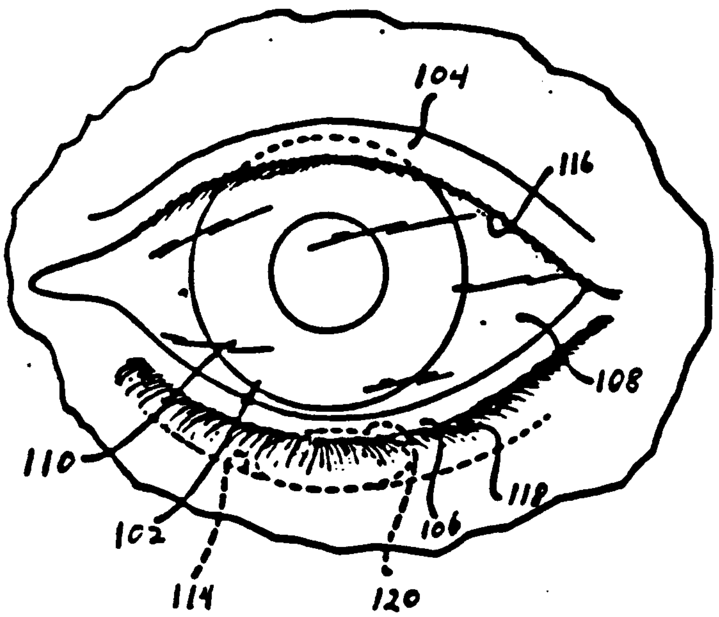


Figure 1

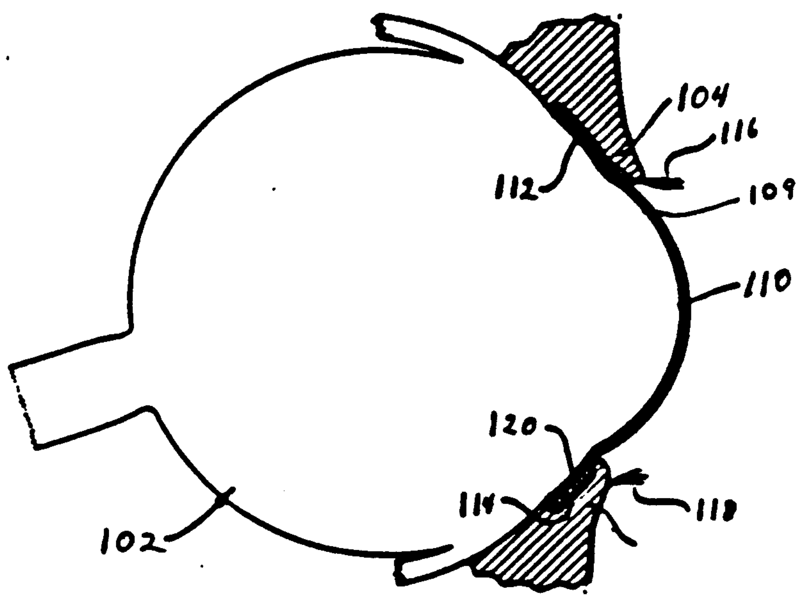


Figure 2

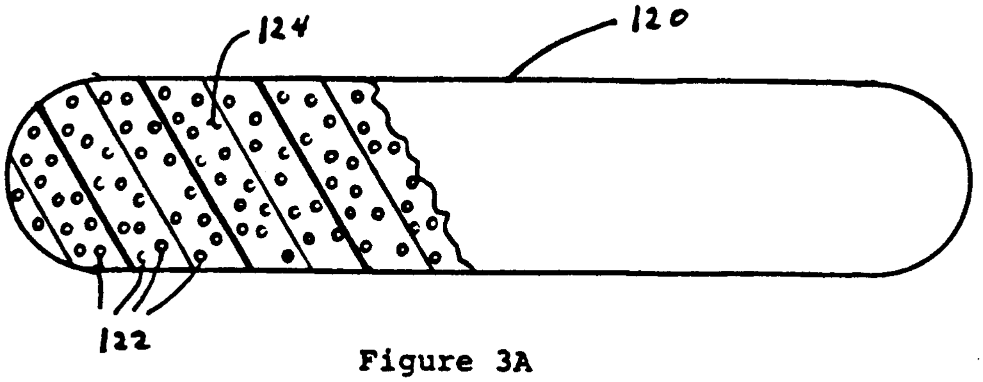


Figure 3A

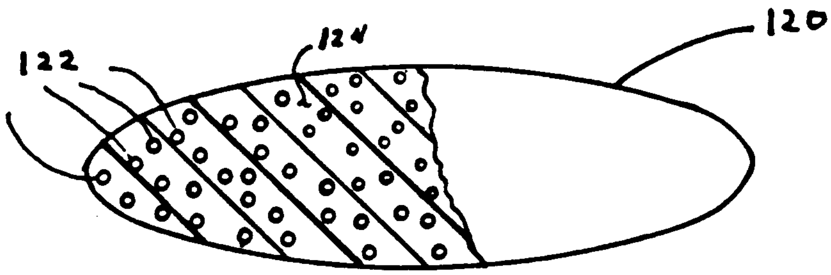


Figure 3B

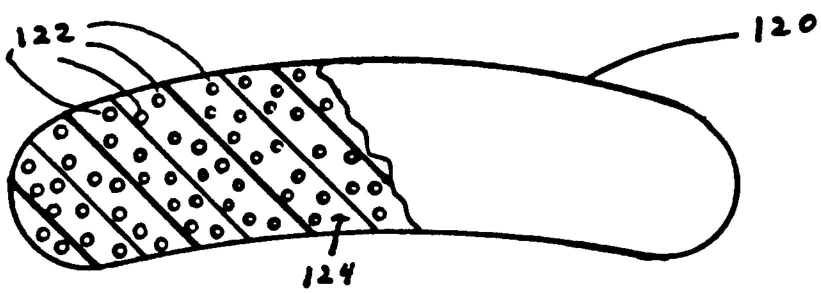


Figure 3C

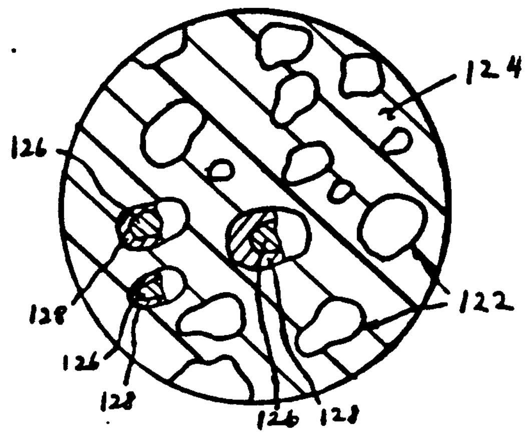


Figure 4

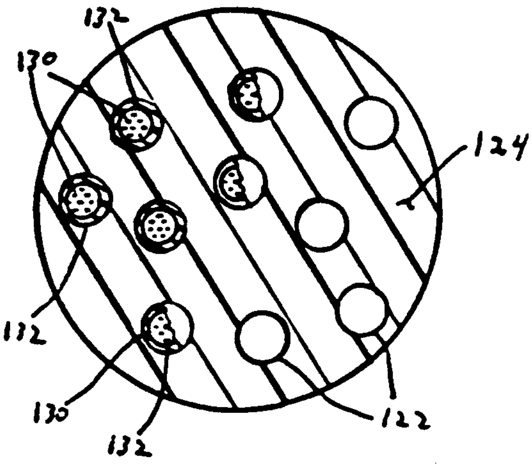


Figure 5

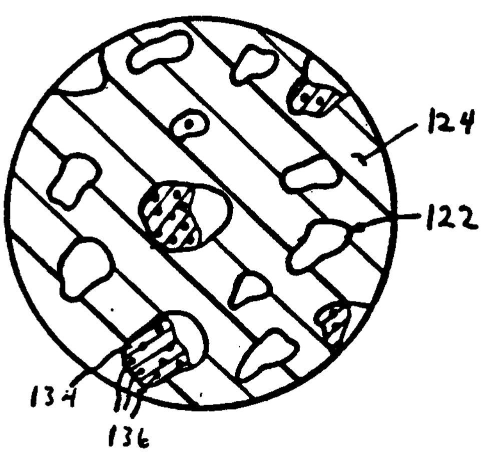


Figure 6

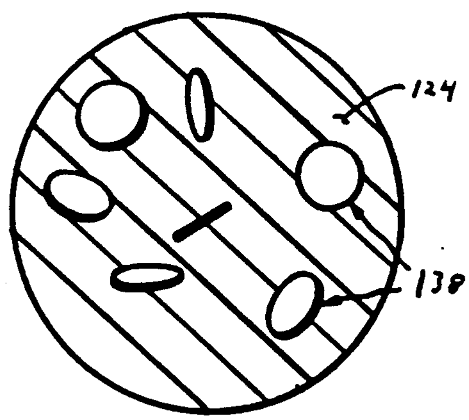


Figure 7

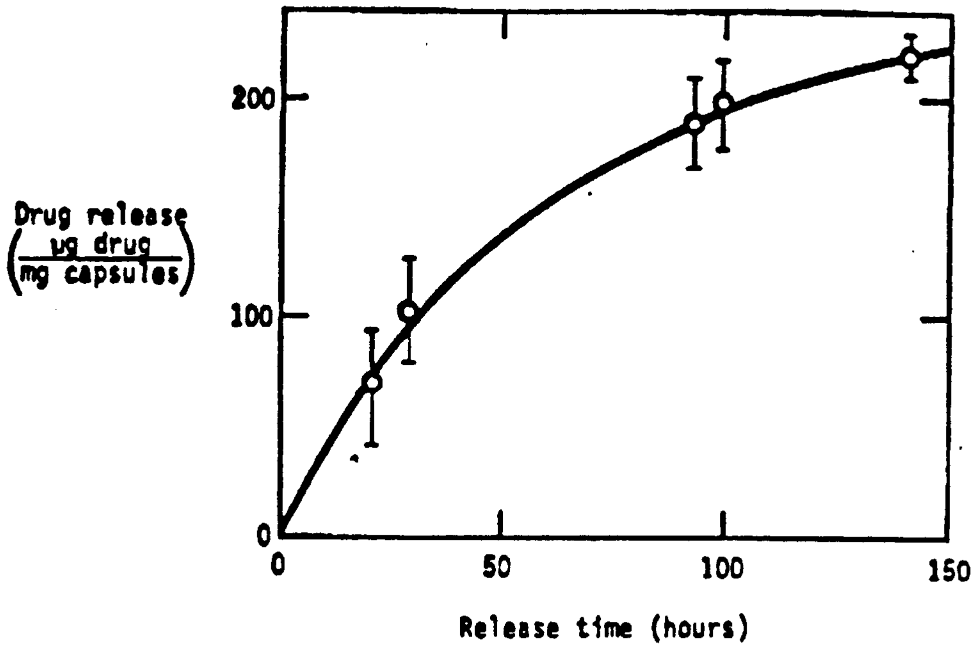


Figure 8

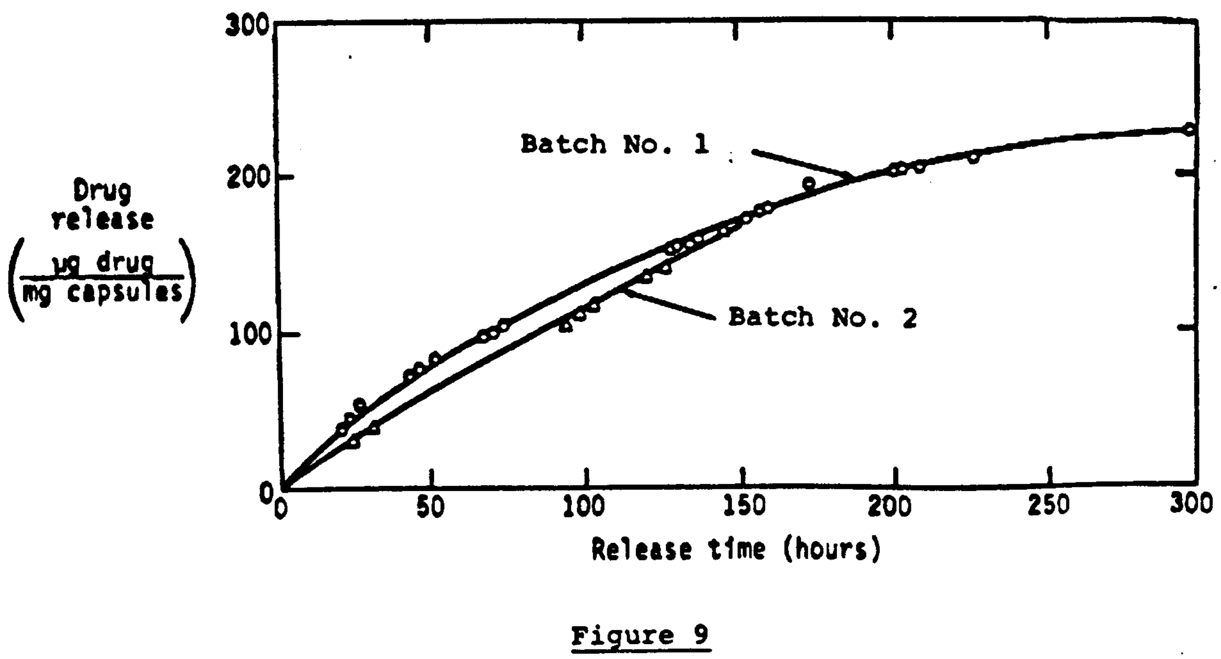


Figure 9